# EUROPEAN PATENT SPECIFICATION

(11) **EP 3 055 418 B1**
(45) Date of publication and mention of the grant of the patent: **03.03.2021**
(21) Application number: 14852274.1
(22) Date of filing: 08.10.2014
(51) Int. Cl.: C12P 7/18, C12P 7/56, C12P 7/52, C12P 19/24, C12P 7/04, C12P 7/40

(54) **METHOD FOR PRODUCING MANNITOL BY USING INTESTINAL BACTERIA FROM SLAUGHTERED ANIMALS**
VERFAHREN ZUR HERSTELLUNG VON MANNITOL DURCH VERWENDUNG VON DARMBAKTERIEN VON SCHLACHTTIEREN
PROCÉDÉ POUR LA PRODUCTION DE MANNITOL EN UTILISANT DES BACTÉRIES INTESTINALES PROVENANT D'ANIMAUX ABATTUS

(30) Priority: 08.10.2013 FI 20130286
(43) Date of publication of application: 17.08.2016
(73) Proprietor: Hakalehto, Eino Elias, 70110 Kuopio (FI)
(72) Inventor: Hakalehto, Eino Elias, 70110 Kuopio (FI)
(74) Representative: Tomkinson, Alexandra
(86) International application number: PCT/FI2014/000026
(87) International publication number: WO 2015/052369

(56) References cited:
- EP-A2- 0 486 024
- WO-A1-00/04181
- WO-A1-02/50296
- WO-A1-80/02282
- WO-A1-2012/118365
- KR-A- 20120 051 961
- US-A- 5 459 053
- US-A1- 2003 215 931
- US-A1- 2011 111 475
- WU, X. ET AL.: 'Continuous biohydrogen production from liquid swine manure supplemented with glucose using an anaerobic sequencing batch reactor.' INTERNATIONAL JOURNAL OF HYDROGEN ENERGY vol. 34, no. 16, 2012, pages 6636 - 6645, XP026496812
- Ralph W. Tyree ET AL: "The production of propionic acid from sugars by fermentation through lactic acid as an intermediate", Journal of Chemical Technology and Biotechnology, vol. 50, no. 2, 1 January 1991 (1991-01-01), pages 157-166, XP055692707, ISSN: 0268-2575, DOI: 10.1002/jctb.280500203

## Description

### Background

Mannitol that forms as a result of bacterial metabolism normally comes from fructose. In nature, fructose is found, e.g. in hemicellulose (for example in wood material), and in various parts of plants, such as in fruits. Wastes and by-products that contain fructose are thus well suitable as raw materials for mannitol production.

Fructose can also be formed from raw materials that contain glucose (cellulose, starch, etc.). For example, the starch from corn, barley, potato, or some other plant, can be split, with the help of amylase and amyloglucosidase enzymes, into glucose, which is then, with the help of glucose- isomerase enzymes, converted into fructose. This is the process used in the production of High-Fructose corn syrup. It has been developed into an industrial viable product and High-Fructose corn syrup is an important sweetener. Correspondingly, different industry sugar fractions can be enzymatically processed and the fructose content and sweetening value can thus be increased.

The broad use of fructose in foodstuffs as a component and sweetener in human nutrition has been hindered lately by the uncertainty of its healthiness. Fructose only breaks down in the body in the liver and excessive use has been shown to cause obesity.

Stomach manure is the content of the stomach that forms in the rumen in bovine and other ruminants. In slaughter houses the amount of stomach manure produced is approximately 50 kg per bovine. Thus, in a slaughterhouse that slaughters 200 animals a day, about 10 tons of stomach manure is gathered per day. Stomach manure is also naturally formed from the slaughtering of other non-bovine animals. The utilization of this waste, for example by composting or by producing biogas, requires considerable space and expensive investment. Thus, the economic benefit and increase of cleaning power gained by using a fast biotechnical process is significant.

Stomach manure microbes exist also in the manure of other slaughter animals. Stomach manure or manure can be combined with other wastes in different waste treatment processes.

US2003/0215931 discloses the production of mannitol in a fermentative method using *Lactobacillus intermedius* from fructose.

EP0496024 discloses the use of a new lactic acid bacteria strain Lactobacillus sp. B001 to produce mannitol.

WO80/02282 discloses the use of manure or slaughterhouse waste to which a lactic acid bacteria is added and a carbohydrate to allow the production of ammonium lactate, a synthetic protein for ruminants.

US5459053 discloses the production of lactic acid using rumen content collected from slaughtered cattle or sheep.

WO2012/118365 discloses a method of producing a bacterial inoculum using rumen of a ruminant animal.

KR20120051961 discloses the use of rumen content to activate the growth of Bacillus and yeast to improve the activity of cellulase and xylanase.

Wu, X. et al. - "Continuous biohydrogen production from liquid swine manure supplemented with glucose using an anaerobic sequencing batch reactor". International Journal of Hydrogen Energy, 2012, Vol. 34, No. 16, S 6636-6645, discloses biohydrogen production from liquid swine manure.

WO02/50296 discloses the use of lactic acid bacteria to produce D-Mannitol from fructose.

### Summary of the Invention

According to the present invention there is provided a method for producing mannitol from fructose present in stomach manure according to claim 1.

### Description

When mannitol is produced using microbes, lactic acid bacteria are normally the production organisms that are used. They are known as safe production organisms. However, it can be challenging to find a suitable strain of organism for the conversion of fructose to mannitol which is efficient.

Using the method according to the present invention, slaughter waste that is rich in fructose, and a by-product or a supplement of fructose, is added into slaughter waste, can produce mannitol. The slaughter waste contains, for example at a bovine slaughterhouse, a large amount of so-called stomach manure (which, in practice, is the content of the rumen and other stomach content of the slaughtered animals) of about 50 kg per animal.

With the help of stomach manure microbes, or other microbes added to them, and also other different waste materials that can be integrated into the process, the method of the present invention can be utilized to produce mannitol.

The microflora of the rumen that is contained in the stomach manure is capable of, with the help of microflora's common metabolism, change fructose that is in the waste, by-product added into it, into mannitol. At the same time, the lactic acid bacteria form lactate which lowers the pH value of the process liquid. With the help of bacteria, such as *Propionibacterium shermalli* and *Propionibacterium acidipropionici*, lactate can be converted into propionate, i.e. propionic acid. The *Clostridium propionicum* bacteria can form propionic acid, acetate, ammonia and carbon dioxide from L-alanine. The optimal pH for the formation of propionic acid is often 6.5. In addition to these products mentioned above, several other organic and inorganic compounds are also formed in the process. The ammonium salts formed in it can be precipitated apart from the process liquid and collected for use as a fertilizer, together with the residue of the process liquid, or it can be separated from it. Thus, using the method according to the present invention, fertilizers may be produced from stomach manure or other waste.

The mixed microbe population or mixed culture of the rumen can also convert glucose found in waste material, by-products or other raw material, into mannitol. This requires the glucose to be first enzymatically converted into fructose. In this case the same glucose-isomerase enzymes that are used in the production of fructose syrup (High Fructose corn syrup (HFCS), Glucose-Fructose syrup (GFS), High-Fructose maize-syrup, glucose/fructose) can be used.

In practice, HFCS is produced from raw material that is rich in starch, mainly from corn. Also, many other plants such as potato and cereal plants contain significant amounts of plant starch. The following enzymes participate in the reaction:
1. Alpha-amylase (forms short chain oligosaccharides from starch)
2. Glucoamylase (splits oligosaccharides into glucose)
3. Glucose-isomerase (converts about 42% of fructose and 50-52% of glucose and some other sugars into the mixture)

Using a method according to the present invention, fructose is produced from glucose with, if necessary, the help of the industrial enzymes mentioned above or with the help of other industrial enzymes that release glucose. For example, with the help of lactic acid bacteria, mannitol can be obtained from fructose. With the help of the process of the present invention, the added value of many biomasses, side streams and waste materials can thus be increased. Glucose is a molecular structure that is commonly present in natural materials, such as starch, cellulose and hemicellulose. Fructose is found for example, in the wastes and side streams of sugar, fruit and berry industries. Naturally, it is clear that in this case microbe cells or other cells, tissue, cell parts or similar may function as biocatalysts, in addition to or along with enzymes or as sections that replace them.

The potential harmfulness of extensive use of fructose has already been raised. In recent discussion related to health, nutrition and chronic illnesses, it is advantageous to biotechnically refine fructose further to another useful form. Contrary to, for example glucose, fructose does not metabolize in all the cells of the body, only in the liver. If excessive amounts of fructose are used this results in, for example, obesity and abdominal obesity risks, and also increases the risk of a fatty liver and other liver diseases. The energy content of mannitol is fairly low compared to fructose and glucose and many other molecules but it has considerable value in its use, such as for example, in lozenges, chewing gums, medicinal products, pastries, and as an additive and sweetener that improves product quality; such as taste, freshness etc. In this respect, there is justification for converting fructose, glucose and other sugars to mannitol.

When sucrose (which consists of glucose and fructose) was added into slaughter waste (stomach manure etc), large amounts of mannitol were formed in it. The pH was not adjusted or kept at a certain pH level, contrary to what is normally advantageous in biotechnical processes, yet the yield was close to the theoretical maximum level, being approximately 18mg/ml. When the pH level was adjusted to between 5 and 6.5, the corresponding production level was only 3mg/ml. The high level of mannitol that occurred when the pH level was not adjusted during the biotechnical process was an unexpected reaction of the normal microflora of the rumen.

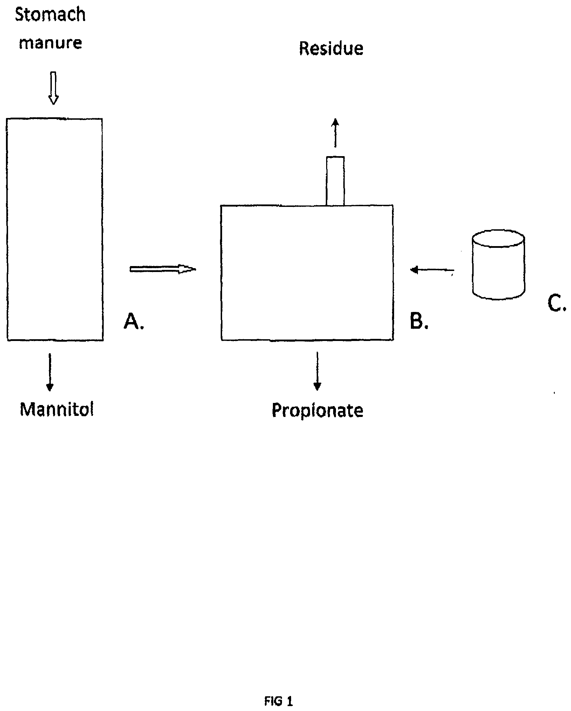

A model for the implementation of the production process of mannitol is presented in Figure 1. If, as described above, the pH is not adjusted or stabilized, considerable amounts of lactate, i.e. lactic acid, formed in the process liquid. An advantageous means of exploiting this is with the help of lactic acid bacteria to convert the lactic acid into propionate, which has a high energy content and which may be used as silage or feed for animals kept for fur, as food for other animals or even as an additive or preservative for human nutrition. It can also be used to prevent the deterioration of timber structures and the icing of road surfaces.

An alternative way to process biomass based suspension or liquid is anaerobic acetone-butanol fermentation. The growth of Clostridia can be accelerated by leading carbon dioxide into the gas solution or process liquid. Since Clostridia can withstand up to 100% carbon dioxide content while growing as a culture, this can be used to speed up the reaction. Clostridia can also be used to produce other organic acids, such as butyric acid and valeric acid.

An installation to exploit the method according to this invention consists of A. a mannitol reactor; B. a production pool for propionic acid formation; and C. a seed fermenter (Figure 1).

## Claims

1. A method for producing mannitol from fructose present in stomach manure, said stomach manure being the content of the rumen from slaughtered bovine and other ruminants, said stomach manure containing fructose, said method including the steps of :
adding sucrose, fructose, glucose or a mixture thereof into the stomach manure;
metabolising the fructose to mannitol with the mixed microflora of the rumen contained in the stomach manure, including lactic acid bacteria,
wherein the lactic acid bacteria form lactate when metabolising fructose to mannitol;
**characterised in that** the method does not include any adjustment to the pH when the fructose is being converted to mannitol.

2. A method according to claim 1, wherein the decomposition of starch, cellulose or hemicellulose by enzymes is used to produce the glucose and/or fructose added to the stomach manure.

3. A method according to any of claims 1-2 **characterized in that** further lactic acid bacteria are added into the stomach manure so as to help the lactic acid bacteria already contained in the stomach manure to convert fructose present in the stomach manure to mannitol.

4. A method according to claim 1 or 3 **characterized in that** a base is added to the stomach manure when all fructose has converted into mannitol in order to raise the pH.

5. A method according to claim 4 **characterized in that** the mannitol is removed from the stomach manure by crystallization prior to raising the pH.

6. A method according to any of claims 1-5 **characterized in that** lactate formed in the stomach manure by the lactic acid bacteria is converted to propionate (propionic acid) *Propionibacterium shermani*, *Propionibacterium acidipropionici* or *Clostridium propionincum.*

## Patentansprüche

1. Verfahren zur Herstellung von Mannitol aus Fructose, die in Magendünger vorhanden ist, wobei der genannte Magendünger der Inhalt des Pansens von geschlachteten Rindern und anderen Wiederkäuern ist, wobei der genannte Magendünger Fructose enthält, wobei das genannte Verfahren die folgenden Schritte beinhaltet:
Zugeben von Saccharose, Fructose, Glucose oder einer Mischung davon zu dem Magendünger;
Metabolisieren der Fructose zu Mannitol mit der im Magendünger enthaltenen gemischten Mikroflora des Pansens, einschließlich Milchsäurebakterien,
wobei die Milchsäurebakterien bei der Metabolisierung von Fructose zu Mannitol Lactat bilden;
**dadurch gekennzeichnet, dass** das Verfahren keine Einstellung des pH-Wertes beinhaltet, wenn die Fructose in Mannitol umgewandelt wird.

2. Verfahren nach Anspruch 1, wobei die Zersetzung von Stärke, Cellulose oder Hemicellulose durch Enzyme zum Produzieren der dem Magendünger zugesetzten Glucose und/oder Fructose verwendet wird.

3. Verfahren nach einem der Ansprüche 1-2, **dadurch gekennzeichnet, dass** dem Magendünger femer Milchsäurebakterien zugegeben werden, um die bereits im Magendünger enthaltenen Milchsäurebakterien bei der Umwandlung der im Magendünger vorhandenen Fructose in Mannitol zu unterstützen.

4. Verfahren nach Anspruch 1 oder 3, **dadurch gekennzeichnet, dass** dem Magendünger eine Base zugesetzt wird, wenn die gesamte Fructose in Mannitol umgewandelt ist, um den pH-Wert anzuheben.

5. Verfahren nach Anspruch 4, **dadurch gekennzeichnet, dass** das Mannitol vor dem Anheben des pH-Werts durch Kristallisation aus dem Magendünger entfernt wird.

6. Verfahren nach einem der Ansprüche 1-5, **dadurch gekennzeichnet, dass** durch die Milchsäurebakterien im Magendünger gebildetes Laktat in Propionat (Propionsäure) *Propionibacterium shermani*, *Propionibacterium acidipropionici* oder *Ciostridium propionincum* umgewandelt wird.

## Revendications

1. Procédé de production de mannitol à partir du fructose présent dans le fumier de contenu stomacal, ledit fumier de contenu stomacal étant le contenu du rumen de bovins et d'autres ruminants abattus, ledit fumier de contenu stomacal contenant du fructose, ledit procédé comprenant les étapes consistant à :
ajouter du saccharose, du fructose, du glucose ou un mélange de ceux-ci dans le fumier de contenu stomacal,
métaboliser le fructose en mannitol avec la microflore mixte du rumen contenu dans le fumier de contenu stomacal, y compris les bactéries productrices d'acide lactique,
où les bactéries productrices d'acide lactique forment du lactate lors de la métabolisation du fructose en mannitol.
**caractérisé en ce que** les bactéries productrices d'acide lactique forment du lactate lorsqu'elles métabolisent le fructose en mannitol ;
**caractérisé en ce que** le procédé ne comprend aucun ajustement du pH lorsque le fructose est converti en mannitol.

2. Procédé selon la revendication 1, dans lequel la décomposition de l'amidon, de la cellulose ou de l'hémicellulose par des enzymes est utilisée pour produire le glucose et/ou le fructose ajouté au fumier de contenu stomacal.

3. Procédé selon l'une des revendications là 2, **caractérisé en ce que** des bactéries productrices d'acide lactique supplémentaires sont ajoutées dans le fumier de contenu stomacal afin d'aider les bactéries productrices d'acide lactique déjà contenues dans le fumier de contenu stomacal à convertir le fructose présent dans le fumier de contenu stomacal en mannitol.

4. Procédé selon la revendication 1 ou 3, **caractérisé en ce qu'**une base est ajoutée au fumier de contenu stomacal lorsque tout le fructose s'est transformé en mannitol afin d'augmenter le pH.

5. Procédé selon la revendication 4, **caractérisé en ce que** le mannitol est éliminé du fumier de contenu stomacal par cristallisation avant que le pH n'augmente.

6. Procédé selon l'une des revendications 1 à 5, **caractérisé en ce que** le lactate formé dans le fumier de contenu stomacal par les bactéries lactiques est converti en propionate (acide propionique) *Priopionibacterium shermani, Propionibacterium acidipropionici ou Clostridium propionincum.*
